# FASCICULE DE BREVET EUROPEEN

(11) **EP 1 439 803 B1**
(45) Date de publication et mention de la délivrance du brevet: **11.11.2015**
(21) Numéro de dépôt: 02793194.8
(22) Date de dépôt: 28.10.2002
(51) Int. Cl.: A61F 9/08, G09B 21/00

(54) **Procédé permettant à au moins un utilisateur, notamment un utilisateur aveugle, de percevoir une forme dans un espace virtuel**
Verfahren, das einem Nutzer, insbesondere einem blinden Nutzer, ermöglicht eine Form in einem virtuellen Raum warzunehmen
Method for enabling at least a user, in particular a blind user, to perceive a shape in a virtual space

(30) Priorité: 26.10.2001 FR 0113897
(43) Date de publication de la demande: 28.07.2004
(73) Titulaire: Université de Technologie de Compiègne, 60206 Compiègne Cedex (FR)
(72) Inventeur: LENAY, Charles, 60200 Compiegne (FR); GAPENNE, Olivier, 76130 Mont Saint-Aignan (FR); HANNETON, Sylvain, 94000 Creteil (FR); MARQUE, Catherine, 60280 Margny-Les-Compiegne (FR); VANHOUTTE, Clotilde, 60200 Compiegne (FR)
(74) Mandataire: Novagraaf Technologies
(86) Numéro de dépôt international: PCT/FR2002/003699
(87) Numéro de publication internationale: WO 2003/034959

(56) Documents cités:
- CH-A- 325 289
- DE-A- 2 538 629
- US-A- 2 327 222
- US-A- 3 229 387
- US-A- 3 594 787
- US-A- 5 956 484
- S Hanneton ET AL: "Dynamique de la reconnaissance de caracteres via une interface haptique", VIIe Colloque de l'Association pour la Recherche Cognitive, ARC'98, December 1997 (1997-12), XP055156046, Paris Retrieved from the Internet: URL:https://hal.archives-ouvertes.fr/docs/ 00/06/29/44/PDF/Hanneton98-DynamiqueDeLaRe connaissance.pdf [retrieved on 2014-12-01]
- Catherine Marque ET AL: "The visual glove", Third International Workshop on Applied Informatics in Biomedicine and Medical Engineering, 1 January 1999 (1999-01-01), XP055156172,
- CHARLES R LENAY ET AL: "Virtual Space and Perception Substitution", PROCEEDINGS OF THE 4TH WORLD MULTICONFERENCE ON SYSTEMICS, CYBERNETICS AND INFORMATICS III (VIRTUAL ENGINEERING AND EMERGENT COMPUTING), 2000, pages 674-677, XP055156114,

## Description

La présente invention se rapporte au domaine de la perception de forme.

La présente invention se rapporte plus particulièrement à un procédé permettant à au moins un utilisateur, notamment un utilisateur aveugle, de percevoir une forme ou une représentation graphique, le procédé utilisant au moins un stimulateur sensoriel.

Pour aider les aveugles à identifier ce qu'ils ne peuvent voir, il est connu d'utiliser la réalisation de relief tactiles ou sonore. En matière tactile, la méthode de base est bien sûr celle développée par M. Braille.

Il a par ailleurs été développé des terminaux brailles composés d'au moins une ligne de cellules brailles, voire de plusieurs lignes de cellules brailles, chaque cellule étant constituée de huit picots, mobiles, chacun, entre une position escamotée et une position saillante. Ces appareils permettent, par exemple, de lire un livre, par interprétation de chaque lettre de chaque mot au fur et à mesure que ces lettres apparaissent sur une cellule.

L'art antérieur connaît également des procédés permettant à des aveugles de percevoir des représentations graphiques, à l'aide d'un stimulateur sensoriel.

Il a par exemple été développé en 1941 un dispositif qui a fait l'objet du brevet américain N° US 2 327 222, délivré en 1943. Ce dispositif comporte un certain nombre de pointes afin de permettre à un aveugle de percevoir une image fixe.

Il a également été développé en 1954 un dispositif qui a fait l'objet du brevet suisse N° CH 325 289, publié en 1957. Ce dispositif a pour but de permettre de reporter à l'aide de picots mobiles, un relief perçu par un ensemble de capteurs.

Actuellement, dans le domaine des stimulateurs tactiles, on arrive à positionner jusqu'à 400 picots mobiles adjacents pour arriver à former un équivalent en relief de la forme captée.

Force est de constater que la démarche actuelle de l'homme du métier est de tenter d'augmenter la résolution des stimulateurs sensoriels, en augmentant le nombre de picots mobiles, afin de permettre de mieux rendre compte des formes. L'inconvénient majeur de cette démarche est qu'elle conduit à réaliser du matériel de plus en plus complexe et de plus en plus coûteux.

En outre, aucun dispositif actuellement conçu ne permet une interactivité de l'utilisateur lui permuttant de se déplacer dans l'espace virtuel perçu.

La présente invention repose sur une démarche en totale opposition avec la démarche actuelle de l'homme du métier.

En effet, plutôt que tenter d'augmenter le nombre de picots, il a été trouvé que, d'une manière surprenante, on pouvait tout à fait utiliser les stimulateurs simples et peu onéreux tels que ceux utilisés pour la lecture de textes écrits en braille, pour permettre de percevoir des formes

Les articles scientifiques suivants montrent des procédés selon le préambule de la revendication 1:
- S. Hanneton, C. Lenay, O. Gapenne, S. Vermandel, C. Marque: "Dynamique de la reconnaissance de caractères via une interface haptique", Vile Colloque de l'Association pour la Recherche Cognitive, ARC'98, décembre 1997, https://hal.archives-ouvertes.fr/docs/00/06/29/44/PDF/Hanneton98-DynamiqueDeLaReconnaissance.pdf
- Catherine Marque, Gapenne Olivier, Hanneton Sylvain, Lenay Charles, Vanhoutte Clothilde: "The visual glove", Third International Workshop on Applied Informatics in Biomedicine and Medical Engineering, 1999
- Charles R Lenay, Hanneton Sylvain, Gapenne Olivier: "Virtual Space and Perception Substitution", Proceedings of the 4th World Multiconference on Systemics, Cybernetics and Informatics III (Virtual Engineering and Emergent Computing), 2000, pages 674-677.

Pour ce faire, la présente invention est du type décrit ci-dessus et elle est définie, dans son acception la plus large par le procédé de la revendication 1.

La forme est une forme définie, de préférence numérisée, illustrant une représentation graphique quelconque, de forme bidimensionnelle ou tridimensionnelle.

Ladite forme peut être réalisée par ledit utilisateur à l'aide d'un outil de dessin virtuel. Avantageusement, un seul outil peut permettre de dessiner et de « lire ».

Dans l'invention, ladite forme est disponible via un réseau de télécommunication du type Internet. Pour ce faire, il est préférable de définir des espaces d'interactions entre les capteurs virtuels et les formes à percevoir. Ces espaces peuvent être bidimensionnels ou tridimensionnels.

Dans cette application, chaque utilisateur est défini dans ou sur ladite forme par un corps-capteur et par un corps-image. Il est en outre préférable de définir des espaces de rencontre structurés (salles virtuelles, déplacements entre des pièces, ...)

Dans une variante de l'invention, lesdits éléments sensitifs étant des picots pour la lecture de texte en braille, mobiles, chacun, entre une position escamotée et une position saillante, le procédé selon l'invention consiste à entraîner lesdits éléments sensitifs chacun dans au moins un état vibrant dans lequel ils oscillent d'une manière régulière entre la position escamotée et la position saillante, afin de permettre de nuancer la perception de ladite forme.

Ledit ou lesdits éléments sensitifs présentent, de préférence, chacun une pluralité d'états vibrants différents, chaque état vibrant étant associé à une variable quantitative.

Lorsque lesdits éléments sensitifs présentent chacun une pluralité d'états vibrants, chaque oscillation est, de préférence, associée à une couleur ou un niveau de gris et/ou une distance.

La configuration dudit capteur virtuel est, due préférence, modifiable par modification de la position relative des champs d'analyse.

Les champs d'analyse du capteur virtuel présentent, de préférence, une forme générale matricielle, séparée ou emboîtée.

Lesdits champs d'analyse présentent, de préférence, chacun une configuration de parallélogramme, de triangle, de cercle, de portion de cercle, de parallélépipède, de pyramide, de sphère ou de portion de sphère.

La présente invention est applicable en utilisant un dispositif pour la mise en oeuvre du procédé selon l'invention, comportant un ordinateur comportant une mémoire dans laquelle ladite forme est contenue, ledit ordinateur étant éventuellement associé à un écran de visualisation, ledit dispositif comportant en outre un moyen de pointage, ainsi qu'au moins un stimulateur sensoriel.

Ledit moyen de pointage et/ou ledit outil de dessin virtuel est ou sont constitué(s), de préférence, d'un stylet associé à une tablette graphique ou d'un manche à balai (joystick) ou d'une boule roulante (trackball) ou d'un vêtement de données de réalité virtuelle.

Ledit stimulateur sensoriel peut être un stimulateur tactile comportant entre 1 et 80 picots et/ou un stimulateur sonore et/ou un stimulateur visuel.

Avantageusement, la présente invention permet ainsi, à l'aide d'un dispositif de stimulation sensorielle simple de percevoir une forme quelconque en la découvrant pas à pas, en la grossissant ou en la diminuant, ...

Avantageusement, également, la présente invention permet à plusieurs utilisateurs de communiquer via un réseau de télécommunication en s'échangeant des formes ou en découvrant ensemble une forme ou une représentation graphique unique, telle que, par exemple, un paysage de synthèse, mis à disposition sur ledit réseau grâce à un serveur. Le procédé permet ainsi de réaliser une véritable interactivité entre malvoyants sur les mêmes images, notamment pour jouer à distance.

On comprendra mieux l'invention à l'aide de la description, faite ci-après à titre purement explicatif, d'un mode de réalisation de l'invention, en référence aux figures annexées :
- la figure 1 illustre un schéma de principe d'un dispositif permettant la mise en oeuvre du procédé ;
- les figures 2a, 2b et 2c illustrent des exemples de composition de capteurs virtuels ;
- la figure 3 illustre un schéma de principe d'un autre dispositif permettant la mise en oeuvre du procédé ;
- les figures 4a, 4b et 4c illustrent trois associations différentes d'un corps-capteur avec un corps-image ;
- la figure 5 illustre un croisement perceptif ; et
- la figure 6 illustre un autre croisement perceptif.

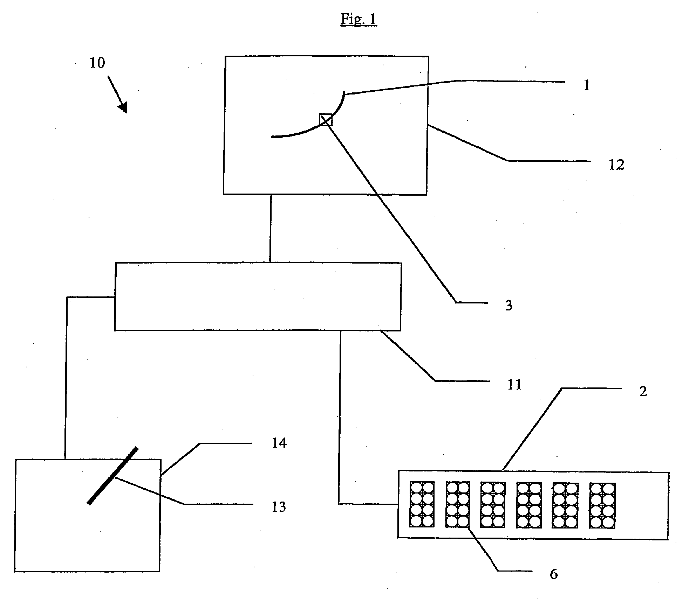

Le procédé selon l'invention, illustré figure 1, est un procédé permettant à au moins un utilisateur, notamment un utilisateur aveugle, de percevoir une forme (1), du type utilisant au moins un stimulateur sensoriel (2).

La forme (1) est une forme définie, de préférence numérisée, illustrant une icône, une image, une représentation graphique quelconque en deux dimensions ou en trois dimensions, ou encore une représentation d'autrui.

Les systèmes de substitution sensorielle pour la perception de formes bidimensionnelles ont montré leur efficacité : les aveugles réussissent après un temps d'apprentissage raisonnable à localiser des objets et événements, et à reconnaître leurs formes.

Toutefois, le coût et la complexité technique de dispositifs de stimulation tactile qui permettent de donner directement à l'utilisateur une perception des formes captées, a représenté un obstacle jusqu'alors insurmontable pour la diffusion de tels systèmes. Le système classique de 400 points distincts de stimulation tactile représente un investissement spécifique et important pour des utilisateurs aveugles.

Au contraire, si l'on ne peut donner que quelques points d'entrée sensorielle tactile à chaque instant, on ne donne plus directement l'information de localisation et/ou de forme.

En outre, si les systèmes existants permettent la perception de formes bidimensionnelles inscrites sur des surfaces planes, ils ne permettent pas en même temps le dessin et l'écriture de formes nouvelles bidimensionnelles et a fortiori tridimensionnelles. Pourtant, la perception et la production (la lecture et l'écriture) de formes bidimensionnelles ou tridimensionnelles a non seulement un intérêt intrinsèque, mais est aussi une méthode utile pour la phase d'apprentissage perceptif.

On définit un capteur virtuel (3) présentant une pluralité de champs d'analyse (4), ledit capteur virtuel (3) étant susceptible d'être déplacé à l'aide d'un moyen de pointage (5) sur ou vers ladite forme (1), chaque champ d'analyse (4) commandant un élément sensitif (6) dudit stimulateur sensoriel (2) afin de permettre de percevoir ladite forme (1) grâce audit stimulateur sensoriel (2) par modification de l'état de chaque élément sensitif (6).

En effet, en donnant les moyens d'une exploration active et rapide de l'espace de perception par un nombre réduit de stimulateurs sensoriels, on compense la faiblesse de cette entrée sensorielle par l'enrichissement de la vitesse et des possibilités de l'action.

L'invention réalise ainsi la mise en relation numérique (via une solution logicielle) entre les actions de l'utilisateur et ses entrées sensorielles. Dès lors on peut utiliser, pour la réalisation du dispositif (10) permettant la mise en oeuvre du procédé, des systèmes de stimulation tactile, visuelle ou auditive économiques et déjà commercialisés.

Le stimulateur sensoriel (2) peut être un stimulateur tactile, comportant par exemple des picots en nombre peu élevés (entre 1 et 80) ou un stimulateur sonore, émettant des sons modulés ou un stimulateur visuel émettant de la lumière à l'aide, par exemple de diodes électroluminescentes, ou encore une combinaison de ces différents stimulateurs.

La forme (1) est contenue dans la mémoire d'un ordinateur (11), éventuellement associé à un écran de visualisation (12). Cette forme (1) est en général mémorisée grâce à ses coordonnées dans le plan ou dans l'espace.

Ledit moyen de pointage (5) et/ou ledit outil de dessin virtuel (7) est ou sont constitué (s) d'un stylet (13) associé à une tablette graphique (14) ou d'un manche à balai (15) ou d'une boule roulante ou d'un vêtement de données de réalité virtuelle, voire simplement d'un clavier ou d'une souris.

Le moyen de pointage (5) permet de commander Les déplacements du capteur virtuel (3) qui permet le contrôle de l'entrée sensorielle de l'utilisateur. Dans le cours de l'activité (mouvements des capteurs commandés par le moyen de pointage (5)), la perception de formes spatiales (localisation et reconnaissance) se constitue dans l'esprit de l'utilisateur.

Le moyen de pointage (5) est le moyen par lequel l'utilisateur commande les déplacements du ou des capteurs virtuels (3). Ces déplacements peuvent être définis en 2 ou 3 dimensions (ou dans des dimensionalités originales).

Il peut y avoir plusieurs moyens de pointage (5) commandant les déplacements de plusieurs capteurs. Les moyens de pointage (5) peuvent permettre de contrôler la forme même des capteurs virtuels (3). De plus, un moyen de pointage (5) peut aussi commander en même temps les déplacements d'un moyen d'inscription modifiant l'espace perçu.

Le capteur virtuel (3) contrôle les stimulations sensorielles en fonction des variations de son environnement et donc essentiellement en fonction de ses déplacements. Un capteur est défini par un ensemble de champs d'analyse (4) qui se déplacent ensemble. Chaque champ d'analyse (4) contrôle un stimulateur sensoriel (2) ou un élément d'un stimulateur sensoriel (2).

L'état du champ d'analyse (4) est défini par l'état de l'environnement couvert par ce champ à la position qu'il occupe. Le nombre, la disposition, les dimensions et formes des champs d'analyse sont paramétrables, soit en début de cession pour une utilisation donnée, soit au cours de l'activité perceptive par l'action du moyen de pointage (5) spécifique.

Les formes perçues ne sont pas données directement et instantanément aux capteurs virtuels et stimulateurs tactiles (ce qui demanderait de pouvoir produire une véritable « image tactile » avec de nombreux points de stimulation). Les formes à percevoir sont définies dans l'environnement numérique par la spécification complète des effets sur les entrées sensorielles de toutes les actions et de tous les enchaînements d'action (tous les déplacements des champs d'analyse commandés par les moyens de pointage (5)). Les formes perçues sont constituées par l'utilisateur lorsqu'il découvre des relations invariantes entre ses actions via le moyen de pointage (5) et ses sensations via les stimulateurs sensoriels. On a pu montrer que ces invariants permettent la constitution d'une perception de la localisation spatiale d'objets et d'événements simples ainsi que la perception de leurs formes spatio-temporelles. En même temps, et par une activité assez semblable à celle de la perception, le même dispositif permet l'inscription, le dessin de formes à percevoir. Il suffit d'ajouter un système de déclenchement d'une fonction écriture contrôlée par l'utilisateur à l'aide d'un outil de dessin virtuel (7).

Sur la figure 1, le moyen de pointage (5) ne commande que des déplacements et rotations du capteur virtuel (3) dans un espace bidimensionnel. La forme (1) à percevoir est comme une forme dessinée dans la surface de l'écran (12) de l'ordinateur (11) et les déplacements du capteur virtuel (3) correspondent aux déplacements du curseur illustrant ce capteur virtuel (3) sur cet écran. Dans cet exemple, les déplacements du curseur sont commandés par le stylet (13) de la tablette graphique (14). Suivant l'état de l'environnement sous les champs d'analyse du capteur virtuel (3), le logiciel commande l'activation des picots de la barrette de cellules brailles électroniques. Ici aussi, les champs d'analyse, leur nombre, formes et dispositions sont paramétrables, soit une fois pour toute pour des applications spécifiques, soit sous le contrôle direct de l'utilisateur (zoom, changement de disposition, ...).

La configuration dudit capteur virtuel (3) est modifiable par modification de la position relative des champs d'analyse (4), à l'aide, par exemple du moyen de pointage (5) ou de tout autre commande.

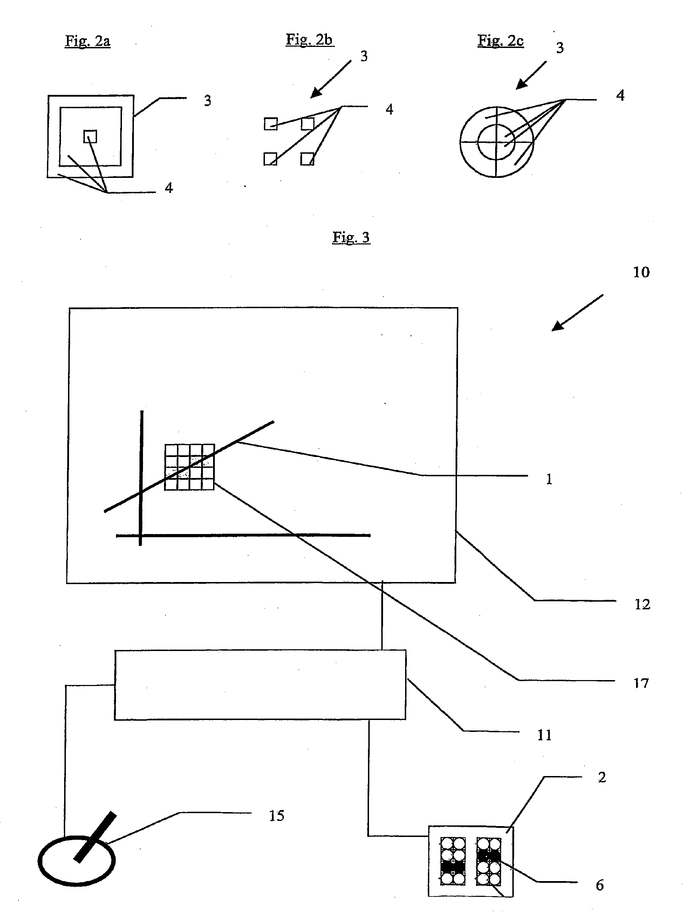

Les champs d'analyse (4) du capteur virtuel (3) présentent une forme générale :
- emboîtée, comme illustré figure 2a avec trois champs d'analyse (4) commandant trois éléments sensitifs (6) distincts et figure 2c avec huit champs d'analyse (4) organisés en secteurs circulaires commandant huit éléments sensitifs (6) distincts, ou
- séparée, comme illustré figure 2b avec quatre champs d'analyse (4) commandant quatre éléments sensitifs (6) distincts ou
- matricielle, comme illustré figure 3.

Les champs d'analyse (4) présentent chacun une configuration en deux dimensions de parallélogramme, de triangle, de cercle, de portion de cercle, ou en trois dimension de parallélépipède, de pyramide, de sphère ou de portion de sphère ou de cas particuliers de celles-ci (rectangle, cube, ...).

Les champs d'analyse sont définis comme ensemble d'éléments tridimensionnels et localisés en x,y,z dans un espace de déplacements.

Les formes à percevoir sont définies comme ensemble d'éléments tridimensionnels et localisées en x,y,z dans ce même espace.

Les stimulations sensorielles sont définies à chaque instant par l'état des intersections entre ces champs d'analyse en déplacement et les formes à percevoir (qui peuvent elles-mêmes être en déplacement).

Donc connaissant en entrée :
- la forme à percevoir, sa position et ses mouvements
- les champs d'analyse, leur position et leurs mouvements,
on a en sortie les stimulations sensorielles à émuler.

Sur la figure 3, le logiciel assure le couplage entre les mouvements du manche à balai (15) ou joystick permettant l'exploration d'un espace, et des stimulations tactiles distribuées en fonction des formes virtuelles rencontrées.

Le manche à balai (15) pilote un capteur permettant d'explorer une forme en noir (ou couleur) sur fond blanc affichée sur l'écran (12) de l'ordinateur (11). Le capteur virtuel (3) correspond à une matrice (17) de champs d'analyse (4) (par exemple une matrice de 16 champs de 3x3 pixels chacun). Quand un champ d'analyse (4) croise au moins un pixel noir, il déclenche l'activation de l'élément sensitif (6) escamoté en l'élément sensitif (6') saillant correspondant sur la cellule de la barrette braille.

Le système de stimulation tactile est exploré par la main libre (l'autre maniant le manche à balai). Il consiste en deux cellules contiguës de 8 picots chacune, ce qui donne une petite matrice de 16 picots sur un peu plus d'1 cm². Cette petite matrice est suffisante pour l'application correspondant à cette réalisation particulière.

Ce dispositif de suppléance perceptive rend ainsi possible l'exploration d'une image tactile numérique. On a montré qu'il permettait la localisation, la reconnaissance et la poursuite de formes à l'aveugle par la simple mise en relation des retours tactiles et des mouvements exploratoires effectués. L'intérêt d'un tel dispositif est que la relation entre les mouvements et les retours tactiles est purement numérique. Elle est donc modifiable et modulable à volonté : non seulement l'image peut changer, mais aussi le nombre, la forme et la disposition des champs d'analyse.

Il est à noter que l'orientation en rotation des champs d'analyse (4) peut être contrôlée. De plus, l'utilisateur aveugle peut mobiliser une fonction de passage en mode écriture pour inscrire des formes qu'il apprendra à reconnaître.

Suivant les systèmes braille commercialisés, le moyen de pointage (5) et les stimulateurs tactiles pourront, soit être intégrés dans un même objet (par exemple, ordinateur portable pour aveugle possédant un mini-manche à balai), soit séparés en deux ou trois éléments indépendants.

Par la mise en relation de ses actions avec leurs retours sensoriels, l'utilisateur aveugle apprendra à reconnaître la position, l'orientation et la forme des courbes explorées.

Dans une variante de l'invention, les éléments sensitifs (6) étant des picots pour la lecture de texte en braille, mobiles, chacun, entre une position escamotée et une position saillante, lesdits éléments sensitifs (6) présentent en outre chacun au moins un état vibrant dans lequel il oscille d'une manière régulière entre la position escamotée et la position saillante, afin de permettre de nuancer la perception de ladite forme (1).

Ledit ou lesdits éléments sensitifs (6) peuvent présenter un état vibrant unique, dans ce cas, ledit ou lesdits éléments sensitifs (6) présentent chacun trois états : deux états fixes et un état intermédiaire oscillant.

Ledit ou lesdits éléments sensitifs (6) peuvent également présenter plusieurs états vibrants différents, chaque état vibrant étant alors associé à une variable quantitative.

Dans ce cas, lesdits éléments sensitifs (6) présentant chacun une pluralité d'états vibrants, chaque oscillation d'un élément sensitif (6) ou d'un ensemble d'éléments sensitifs (6) est alors associée, selon les cas, à une couleur ou un niveau de gris et/ou une distance.

Tous les systèmes d'assistance perceptive ont été développés pour des handicapés sensoriels isolés. Ils leur donnent, par exemple aux aveugles, à percevoir des éléments du monde des voyants, mais ne traitent pas de la question de l'interaction à distance de différents utilisateurs aveugles, interaction sur leurs positions respectives, et sur des inscriptions partagées dans un espace commun.

De plus, les systèmes de substitution sensorielle classiques prétendent donner aux aveugles une perception ressemblant à la vision. Ils ne prennent pas la mesure de l'originalité propre de la modalité perceptive nouvelle qu'ils produisent. Au contraire, en reconnaissant l'originalité de l'expérience perceptive créée, on peut concevoir de nouvelles utilisations de ces systèmes pour un public plus large que celui des handicapés sensoriels.

Enfin, les systèmes de réalité virtuelle actuels, peuvent assurer des rencontres entre les corps-images (avatars) de plusieurs utilisateurs, mais ils ne permettent pas de véritables croisements de regard parce qu'il n'y a pas croisements des activités perceptives elles-mêmes.

Ainsi, dans l'invention, ladite forme (1) est disponible via un réseau de télécommunication du type Internet.

Les systèmes dédiés à la perception active de formes spatiales dans les espaces numériques (i.e. espaces virtuels) permettent en même temps la mise en place d'un dispositif d'interaction original. Dans la mesure ou ces systèmes d'interface rendent possible la construction active de perceptions spatiales, ils permettent en même temps la construction d'espaces virtuels de rencontre perceptive. La mise en réseau (soit par câblage direct, soit via le réseau Internet) de plusieurs de ces systèmes permet de créer des espaces de rencontre et d'interaction perceptive : la maçon dont, pour chaque utilisateur, ses entrées sensorielles sont définies en fonction de ses déplacements dans un espace de perception, dépend de la position et de la forme du corps d'autres utilisateurs présents dans ce même espace. Chaque utilisateur est défini dans le système par un corps-capteur (18) et par un corps-image (19).

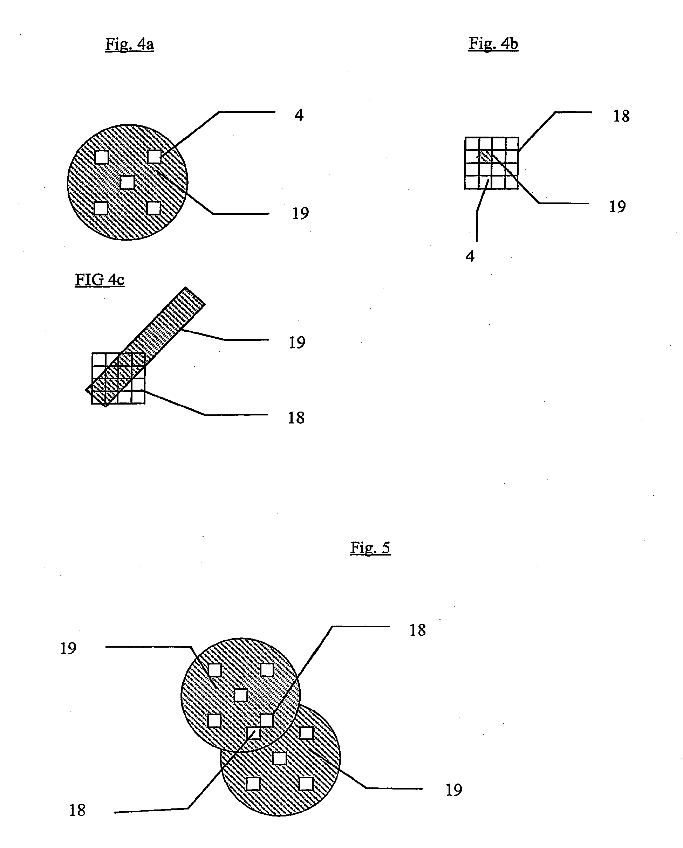

Le corps-capteur (18), illustré figure 4a, correspond à l'organisation des champs d'analyse (4) dont l'utilisateur commande les déplacements via le moyen de pointage (5) (souris, clavier, joystick, stylet de tablette graphique, etc.), c'est-à-dire, à une matérialisation du capteur virtuel. Les stimulations sensorielles délivrées à chaque instant à l'utilisateur sont contrôlées par l'état de l'environnement à cet instant à l'emplacement de ces champs d'analyse. En général, un champ d'analyse (4) est lié à un élément sensitif, et il y a donc autant de champs d'analyse (4) que d'éléments sensitifs. L'utilisateur peut choisir et moduler le nombre, la disposition et la forme de ses champs d'analyse (4). Par l'exploration active de l'espace virtuel dans lequel il déplace ses champs d'analyse (4), l'utilisateur découvre des relations invariantes entre ses actions (ses déplacements commandés par les moyens de pointage (5)) et ses sensations (les stimulations sensorielles qu'il reçoit). Ce sont ces relations invariantes qui permettent à l'utilisateur de constituer la perception (localisation et reconnaissance de forme) d'objets et événements dans cet espace virtuel.

Le corps-image (19), illustré figure 4b, se déplace avec le corps-capteur (18). Il définit l'état de l'environnement à sa position pour les champs d'analyse d'autres utilisateurs qui partagent le même espace virtuel d'interaction. Le corps-image d'un utilisateur est donc une forme en déplacement, potentiellement perceptible par d'autres utilisateurs. Chaque utilisateur peut définir la forme de son corps-image. Elle peut être plus grande, plus petite, superposée ou décalée par rapport au corps-capteur (18) défini par les champs d'analyse (4), comme illustré figure 4c. On peut aussi définir un corps-image non symétrique dans toutes les directions de l'espace et dont l'utilisateur contrôle l'orientation. Ce contrôle peut, par exemple, être réalisé avec pour moyen de pointage (5), comme par exemple un stylet d'une tablette graphique qui donne « l'effet tilt », c'est-à-dire l'orientation et l'inclinaison du stylet.

Si les corps-capteur et corps-image des différents utilisateurs sont suffisamment semblables, quand des champs d'analyse (4) du corps-capteur (18) d'un utilisateur recouvrent le corps-image (19') d'un autre utilisateur, simultanément et réciproquement, les champs d'analyse (4) du corps-capteur (18') de ce deuxième utilisateur recouvrent le corps-image (19) du premier utilisateur, comme illustré figure 5.

Dans cette situation, les corps-capteurs et les corps-images étant semblables et symétriques, quand des champs d'analyse du corps-capteur (18) d'un utilisateur recouvrent le corps-image (19') d'un autre utilisateur, les champs d'analyse de ce dernier recouvrent le corps-image (19) du premier : on ne peut voir sans être vu.

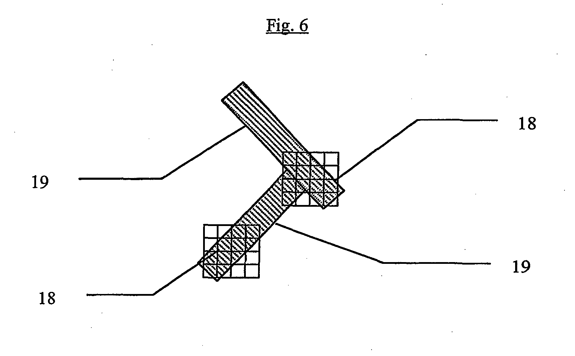

Au contraire si les corps-images ou les champs d'analyse sont asymétriques et orientables, il est possible que le corps-capteur (18) d'un utilisateur recouvrent le corps-image (19') d'un autre utilisateur sans que le corps-capteur (18') de ce dernier ne recouvrent le corps-image (19) du premier : on verra sans être vu, comme illustré figure 6.

L'intérêt de ces croisements perceptifs est que, dans le cours de l'activité perceptive des différents utilisateurs, chacun peut reconnaître non seulement la présence d'une forme en déplacement, mais que cette forme est celle d'un autre utilisateur percevant, et non pas une forme passive inscrite dans l'environnement. Ce qui est donné à percevoir dans un tel système ce ne sont pas seulement des choses ou même des corps, mais aussi l'activité perceptive d'autrui elle-même, et ceci dans la situation très particulière où cette activité est orientée vers la perception de la propre activité perceptive de l'utilisateur. On réalise ainsi, dans une modalité perceptive très particulière une forme de « croisement de regard » ou de « caresse mutuelle » à distance. En effet, si, comme dans l'application exemplaire détaillée ci-dessous, la stimulation sensorielle est de type tactile, on peut dire que l'on produit ainsi une forme de toucher à distance dans des espaces virtuels de rencontre tactile.

La condition générale de la réussite de ces interactions perceptives est la vitesse de transmission des informations dans le réseau qui lie les différents utilisateurs via leurs interfaces perceptives. Cependant, cette grande vitesse peut aisément être atteinte parce que la quantité d'information qui transite à chaque instant est très faible. Les formes des corps-capteurs et corps-images des différents utilisateurs sont transmises en début de session et n'ont besoin d'être réactualisées que de temps en temps. Ne doivent transiter rapidement que les informations sur la position des différents corps (x,y,z si l'espace virtuel est tridimensionnel), et suivant le cas, des informations sur l'orientation des corps et/ou leurs possibles déformations.

Les espaces d'interaction peuvent être structurés.

On peut en tracer des limites. Par exemple, le bord d'un espace sera défini par une forme pleine continue qui sature les stimulateurs sensoriels.

On peut autoriser les utilisateurs à écrire dans l'espace partagé pour se donner mutuellement à percevoir des formes. Une part importante de l'apprentissage de l'usage de telles interfaces passe par des séances d'écriture et de lecture des formes écrites. Ceci pourrait aussi par exemple servir pour un enseignement à distance des mathématiques pour les aveugles.

Par ailleurs, l'espace de rencontre peut être, soit un espace collectif où peuvent interagir un grand nombre de utilisateurs, soit se diviser en espace plus restreints où se donnent rendez-vous quelques utilisateurs.

Le logiciel permettant de mettre en oeuvre le procédé selon l'invention peut s'adresser aux jeunes aveugles en classe de mathématique ou pour tout autre enseignement où il est nécessaire de faire comprendre des courbes et des graphiques. Mais bien sûr, il est également utile pour tout le public aveugle qui veut lire des graphiques et courbes et plus généralement identifier des formes, des images, voire des paysages. Il peut facilement être intégré aux systèmes déjà existants.

L'invention est décrite dans ce qui précède à titre d'exemple. Il est entendu que l'homme du métier est à même de réaliser différentes variantes de l'invention sans pour autant sortir du cadre du brevet défini par les revendications.

## Revendications

1. Procédé permettant à au moins un utilisateur, notamment un utilisateur aveugle, de percevoir une forme (1), utilisant au moins un stimulateur sensoriel (2) présentant des éléments sensitifs (6),et tel que :
- l'on définit un espace virtuel dans lequel sont définies des formes;
- l'on définit dans cet espace virtuel un corps-capteur (18) présentant une pluralité de champs d'analyse (4),
- l'on commande le déplacement dudit corps-capteur dans ledit espace virtuel à l'aide d'un moyen de pointage (5) sur ou vers ladite forme, chaque champ d'analyse du corps-capteur commandant un élément sensitif dudit stimulateur sensoriel afin de permettre de percevoir ladite forme (1) grâce audit stimulateur sensoriel (2) par modification de l'état de chaque élément sensitif (6)
**caractérisé en ce que** l'espace virtuel est partagé via un réseau de télécommunication du type Internet, et chaque utilisateur est défini, dans l'espace virtuel, par ledit corps-capteur (18) et par un corps- image (19) constituant une forme déplaçable perceptible par d'autres utilisateurs, chaque utilisateur commandant le déplacement de son corps-image (19) et de son corps-capteur (18) dans l'espace virtuel à l'aide du moyen de pointage sur ou vers la forme constituée du corps-image (19) d'un autre utilisateur.

2. Procédé selon la revendication 1, **caractérisé en ce qu'**une forme (1) est réalisée par ledit utilisateur à l'aide d'un outil de dessin virtuel (7).

3. Procédé selon l'une quelconque des revendications 1 à 2, lesdits éléments sensitifs (6) étant des picots pour la lecture de texte en braille, mobiles, chacun, entre une position escamotée et une position saillante, **caractérisé en ce qu'**il consiste à entraîner lesdits éléments sensitifs (6) chacun dans au moins un état vibrant dans lequel ils oscillent d'une manière régulière entre la position escamotée et la position saillante, afin de permettre de nuancer la perception de ladite forme (1).

4. Procédé selon l'une quelconque des revendications 1 à 3, **caractérisé en ce que** ledit ou lesdits éléments sensitifs (6) présentent chacun une pluralité d'états vibrants différents, chaque état vibrant étant associé à une variable quantitative.

5. Procédé selon la revendication 3 ou la revendication 4, **caractérisé en ce que** lesdits éléments sensitifs (6) présentant chacun une pluralité d'états vibrants, chaque oscillation est associée à une couleur ou un niveau de gris et/ou une distance.

6. Procédé selon l'une quelconque des revendications 1 à 5, **caractérisé en ce que** la configuration dudit corps-capteur est modifiable par modification de la position relative des champs d'analyse (4).

7. Procédé selon l'une quelconque des revendications 1 à 6, **caractérisé en ce que** le nombre, la forme et la disposition des champs d'analyse sont modifiables.

8. Procédé selon l'une quelconque des revendications 1 à 7, **caractérisé en ce que** les champs d'analyse (4) du corps-capteur présentent une conformation générale matricielle, séparée ou emboîtée.

9. Procédé selon l'une quelconque des revendications 1 à 8, **caractérisé en ce que** lesdits champs d'analyse (4) présentent chacun une configuration de parallélogramme, de triangle, de cercle, de portion de cercle, de parallélépipède, de pyramide, de sphère ou de portion de sphère.

## Patentansprüche

1. Verfahren, das es zumindest einem Benutzer, vor allem einem blinden Benutzer, ermöglicht, eine Form (1) wahrzunehmen, indem er zumindest einen sensorischen Stimulator (2) verwendet, der senstive Elemente (6) aufweist, und derart ausgeführt ist, dass:
- man einen virtuellen Raum definiert, in dem Formen definiert sind;
- man in diesem virtuellen Raum einen Sensorkörper (18) definiert, der eine Vielzahl von Analysefeldern (4) aufweist,
- man die Verschiebung des besagten Sensorkörpers im besagten virtuellen Raum mithilfe eines Zeigemittels (5) auf oder zur besagten Form steuert, wobei jedes Analysefeld des Sensorkörpers ein senstives Element des besagten sensorischen Stimulators ansteuert, um die besagte Form (1) dank des besagten sensorischen Stimulators (2) durch die Änderung des Zustands eines jeden senstiven Elements (6) wahrnehmen zu können
**dadurch gekennzeichnet, dass** der virtuelle Raum über ein Telekommunikationsnetzwerk in der Art des Internets gemeinsam genutzt wird, und jeder Benutzer im virtuellen Raum durch den besagten Sensorkörper (18) und durch einen Bildkörper (19) definiert wird, die eine verschiebbare Form bilden, die von anderen Benutzern wahrgenommen werden kann, wobei jeder Benutzer die Verschiebung seines Bildkörpers (19) und seines Sensorkörpers (18) im virtuellen Raum mithilfe des Zeigemittels auf oder zur besagten Form steuert, die vom Bildkörper (19) eines anderen Benutzers gebildet wird.

2. Verfahren nach Anspruch 1, **dadurch gekennzeichnet, dass** eine Form (1) durch den besagten Benutzer mithilfe eines virtuellen Zeichenwerkzeugs (7) erzeugt wird.

3. Verfahren nach irgendeinem der Ansprüche 1 bis 2, wobei die besagten senstiven Elemente (6) Spitzen zum Lesen eines Textes in Blindenschrift sind, die jeweils zwischen einer eingezogenen Position und einer überstehenden Position bewegt werden können, **dadurch gekennzeichnet, dass** es darin besteht, die besagten senstiven Elemente (6) jeweils zuindest in einen schwingenden Zustand zu versetzen, in dem sie regelmäßig zwischen der eingezogenen Position und der überstehenden Position schwingen, um die Wahrnehmung der besagten Form (1) zu nuancieren.

4. Verfahren nach irgendeinem der Ansprüche 1 bis 3, **dadurch gekennzeichnet, dass** das oder die besagten senstiven Elemente (6) jeweils eine Vielzahl von unterschiedlichen schwingenden Zuständen aufweisen, wobei jeder schwingende Zustand einer quantitativen Variablen zugeordnet wird.

5. Verfahren nach Anspruch 3 oder nach Anspruch 4, **dadurch gekennzeichnet, dass**, da die besagten senstiven Elemente (6) jeweils eine Vielzahl von schwingenden Zuständen aufweisen, jede Schwingung einer Farbe oder einer Graustufe und/ oder einer Distanz zugeordnet wird.

6. Verfahren nach irgendeinem der Ansprüche 1 bis 5, **dadurch gekennzeichnet, dass** die Konfiguration des besagten Sensorkörpers durch die Änderung der Position der Analysefelder (4) zueinander geändert werden kann.

7. Verfahren nach irgendeinem der Ansprüche 1 bis 6, **dadurch gekennzeichnet, dass** die Anzahl, die Form und die Anordnung der Analysefelder geändert werden können.

8. Verfahren nach irgendeinem der Ansprüche 1 bis 7, **dadurch gekennzeichnet, dass** die Analysefelder (4) des Sensorkörpers eine im Allgemeinen matrixförmige, getrennte oder verschachtelte Gestaltung aufweisen.

9. Verfahren nach irgendeinem der Ansprüche 1 bis 8, **dadurch gekennzeichnet, dass** die besagten Analysefelder (4) jeweils eine Parallelogramm-, Dreiecks-, Kreis-, Kreisabschnitt-, Parallelepiped-, Pyramiden-, Kugel- oder Kugelabschnitt-Konfiguration aufweisen.

## Claims

1. Method for enabling at least one user, in particular a blind user, to perceive a shape (1), using at least one sensory stimulator (2) having sensitive elements (6), and such that:
- a virtual space is defined wherein shapes are defined;
- in this virtual space a sensor body (18) is defined having a plurality of analysing fields (4),
- the displacement of said sensor body in said virtual space is controlled using a pointer means (5) on or towards said shape, with each analysing field of the sensor body controlling a sensitive element of said sensory stimulator so as to enable perception of said shape (1) thanks to said sensory stimulator (2) through modification of the state of each sensitive element (6)
**characterised in that** the virtual space is shared via a telecommunications network of the Internet type, and each user is defined, in the virtual space, by said sensor body (18) and by an image body (19) constituting a shape that can be displaced and that can be perceived by other users, with each user controlling the displacement of his image body (19) and of his sensor body (18) in the virtual space using the pointer means on or towards the shape constituted of the image body (19) of another user.

2. Method according to claim 1, **characterised in that** a shape (1) is made by said user using a virtual drawing tool (7).

3. Method according to any of claims 1 to 2, said sensitive elements (6) being raised dots for the reading of Braille text, mobile, each one, between a retracted position and a protruding position, **characterised in that** it consists in driving said sensitive elements (6) each one in at least one vibrating state wherein they oscillate in a regular manner between the retracted position and the protruding position, in order to enable nuancing the perception of said shape (1).

4. Method according to any of claims 1 to 3, **characterised in that** said sensitive element or elements (6) each have a plurality of different vibrating states, each vibrating state being associated with a quantitative variable.

5. Method according to claim 3 or claim 4, **characterised in that** said sensitive elements (6) each having a plurality of vibrating states, each oscillation is associated with a colour or a level of grey and/or a distance.

6. Method according to any of claims 1 to 5, **characterised in that** the configuration of said sensor body can be modified by changing the relative position of the analysing fields (4).

7. Method according to any of claims 1 to 6, **characterised in that** the number, the shape and the arrangement of the analysing fields can be modified.

8. Method according to any of claims 1 to 7, **characterised in that** the analysing fields (4) of the sensor body have a general matrix, separated or nested conformation.

9. Method according to any of claims 1 to 8, **characterised in that** said analysing fields (4) each have a configuration of a parallelogram, triangle, circle, portion of a circle, parallelepiped, pyramid, sphere or portion of a sphere.
